# EUROPEAN PATENT APPLICATION

(11) **EP 1 616 955 A1**
(43) Date of publication of application: **18.01.2006**
(21) Application number: 04724744.0
(22) Date of filing: 31.03.2004
(51) Int. Cl.: C12N 15/56, C12N 15/60, C12N 9/26, C12N 9/88, A61K 38/47

(54) **CATALYST FOR SUGAR CHAIN CLEAVAGE**

(30) Priority: 31.03.2003 JP 2003097301; 18.04.2003 JP 2003113965
(71) Applicant: SEIKAGAKU CORPORATION, Chiyoda-ku, Tokyo 100-0005 (JP)
(72) Inventor: TANAKA, Masayuki, Kuwana-shi, Mie511-0935 (JP); KYOGASHIMA, Mamoru, Higashiyamato-shi, Tokyo 207-0023 (JP)
(74) Representative: HOFFMANN EITLE
(86) International application number: PCT/JP2004/004695
(87) International publication number: WO 2004/097022

(57) **Abstract**

A catalyst which is capable of specifically cleaving one sugar chain or two or more sugar chains selected from the group consisting of hyaluronic acid, chondroitin sulfate A, chondroitin sulfate C and chondroitin sulfate D; a cleavage agent specific for the sugar chains; a medicament which is used for specifically cleaving the sugar chains in a living body tissue; a method for specifically cleaving the sugar chains; a method for specifically producing the sugar chain having a decreased molecular weight; and production methods for the catalyst, the cleavage agent and the medicament.

## Description

### TECHNICAL FIELD

The present invention relates to a novel catalyst for cleaving a sugar chain, an agent for specifically cleaving sugar chains, and the like.

### BACKGROUND ART

Firstly, abbreviations commonly used in the present specification are described.
HA: hyaluronic acid
CH: chondroitin
CS: chondroitin sulfate
CSA: chondroitin sulfate A
CSC: chondroitin sulfate C
CSD: chondroitin sulfate D
DS (also referred to as CSB): dermatan sulfate
GPC: gel permeation chromatography
KS: keratan sulfate

In this connection, CSA is a molecule wherein a "disaccharide unit in which a glucuronic acid residue and an N-acetylgalactosamine residue are bound via β1,3 glycosidic linkage" is continuously bound, and is CS which comprises a disaccharide unit consisting of "a glucuronic acid residue (β1,3) an N-acetylgalactosamine residue in which the C4-position of the N-acetylgalactosamine residue is sulfated" as the main constituting component.

CSC is a molecule wherein a "disaccharide unit in which a glucuronic acid residue and an N-acetylgalactosamine residue are bound via β1,3 glycosidic linkage" is continuously bound, and is CS which comprises a disaccharide unit consisting of "a glucuronic acid residue (β1,3) an N-acetylgalactosamine residue in which the C6-position of the N-acetylgalactosamine residue is sulfated" as the main constituting component.

CSD is a molecule wherein a "disaccharide unit in which a glucuronic acid residue and an N-acetylgalactosamine residue are bound via β1,3 glycosidic linkage" is continuously bound, and is CS which comprises a disaccharide unit consisting of "a glucuronic acid residue in which the C2-position is sulfated (β1,3) an N-acetylgalactosamine residue in which the C6-position is sulfated" as the main constituting component.

HYAL1 as one kind of hyaluronidase is described in *The Journal of Biological Chemistry*, Vol. 277, pp. 33654-33663 (2002). In addition, a full amino acid sequence of HYAL1 and a full nucleotide sequence of cDNA encoding it are disclosed in *Biochemical and Biophysical Research Communications*, 236(1), pp. 10-15 (1997).

However, it is not known that HYAL1 does not substantially have activity capable of cleaving CSB, KS, and CH having an average molecular weight of 7,000, but has activity capable of cleaving CSA, CSC and CSD.

### DISCLOSURE OF THE INVENTION

An object of the present invention is to provide a novel catalyst for cleaving a sugar chain, which comprises HYAL1 as an essential component, an agent for specifically cleaving sugar chains and the like.

In order to solve the above-described problems, the present inventors have conducted intensive studies and provided a novel catalyst for cleaving a sugar chain, which comprises HYAL1 as an essential component; an agent and a medicament for specifically cleaving a sugar chain; a method for specifically cleaving a sugar chain, which comprises reacting HYAL1 with a specific sugar chain; a method for specifically producing a sugar chain having a decreased molecular weight, which comprises reacting HYAL1 with a specific sugar chain; a method for producing the above-described catalyst, which comprises at least the step expressing a protein using a DNA encoding HYAL1 and collecting the expressed protein; a method for producing the above-described cleavage agent, which comprises at least the step expressing a protein using a DNA encoding HYAL1 and collecting the expressed protein; a method for producing the above-described medicament, which comprises at least the step expressing a protein using a DNA encoding HYAL1 and collecting the expressed protein; and the like.

The present invention provides a catalyst (hereinafter referred to as "catalyst of the present invention"), which comprises the following protein (A) or (B) as an essential component and is capable of specifically cleaving one sugar chain or two or more sugar chains selected from the group consisting of HA, CSA, CSC and CSD:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

It is preferable that the catalyst of the present invention is not capable of cleaving DS, KS, and CH having an average molecular weight of 7,000.

The present invention also provides a cleavage agent (hereinafter referred to as "cleavage agent of the present invention"), which comprises the following protein (A) or (B) as an essential component and is specific for cleavage of one sugar chain or two or more sugar chains selected from the group consisting of HA, CSA, CSC and CSD:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

It is preferable that the cleavage agent of the present invention is not capable of cleaving DS, KS, and CH having an average molecular weight of 7,000.

The present invention also provides a medicament (hereinafter referred to as "medicament of the present invention"), which comprises the following protein (A) or (B) as an essential component and is used for specifically cleaving one sugar chain or two or more sugar chains selected from the group consisting of HA, CSA, CSC and CSD which are present in a living body tissue:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

It is preferable that the medicament of the present invention is not capable of cleaving DS, KS, and CH having an average molecular weight of 7,000.

Also, it is preferable that the "living body tissue" in which the sugar chains to be cleaved by the medicament of the present invention are present is nucleus pulposus. In addition, it is preferable that the medicament of the present invention is an agent for treating disc herniation.

The present invention also provides a method for specifically cleaving one sugar chain or two or more sugar chains selected from the group consisting of HA, CSA, CSC and CSD (hereinafter referred to as "cleavage method of the present invention"), which comprises reacting the following protein (A) or (B) with the sugar chain(s):
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

It is preferable that DS, KS, and CH having an average molecular weight of 7,000 are not cleaved by the cleavage method of the present invention.

The present invention also provides a method for specifically producing a sugar chain(s) having a decreased molecular weight (hereinafter referred to as "production method of the sugar chain of the present invention"), which comprises reacting the following protein (A) or (B) with one sugar chain or two or more sugar chains selected from the group consisting of HA, CSA, CSC and CSD:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

It is preferable that DS, KS and CH having a decreased molecular weight are not produced by the production method of the sugar chain of the present invention.

The present invention also provides a method for producing the catalyst of the present invention (hereinafter referred to as "production method of the catalyst of the present invention"), which comprises expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

The present invention also provides a method for producing the cleavage agent of the present invention (hereinafter referred to as "cleavage agent producing method of the present invention"), which comprises expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

The present invention also provides a method for producing the medicament of the present invention (hereinafter referred to as "medicament producing method of the present invention"), which comprises expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

The present invention further provides a fusion protein (hereinafter referred to as "fusion protein of the present invention"), which comprises the following protein (A) or (B) with other peptide:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

It is preferable that the fusion protein of the present invention is not capable of cleaving DS, KS, and CH having an average molecular weight of 7,000.

In addition, the present invention provides a method for treating a disease in which one sugar chain or two or more sugar chains selected from the group consisting of HA, CSA, CSC and CSD is/are excessively present in a living body tissue (hereinafter referred to as "treating method of the present invention"), which comprises administering the above-described medicament of the present invention.

A preferable medicament of the present invention and a preferable living body tissue in the treating method of the present invention are the same as those in the above-described medicament of the present invention.

### BRIEF DESCRIPTION OF THE DRAWINGS

Fig. 1 is a graph showing a result when HA was used as the substrate of HYAL1.
Fig. 2 is a graph showing a result when CH was used as the substrate of HYAL1.
Fig. 3 is a graph showing a result when CSA was used as the substrate of HYAL1.
Fig. 4 is a graph showing a result when CSB was used as the substrate of HYAL1.
Fig. 5 is a graph showing a result when CSC was used as the substrate of HYAL1.
Fig. 6 is a graph showing a result when CSD was used as the substrate of HYAL1.
Fig. 7 is a graph showing a result when KS was used as the substrate of HYAL1.
Fig. 8 is a graph showing a result of chromatography when HYAL1 was used as the enzyme source, and HA was used as the substrate.
Fig. 9 is a graph showing a result of chromatography when HYAL1 was used as the enzyme source, and CSA was used as the substrate.
Fig. 10 is a graph showing a result of chromatography when HYAL1 was used as the enzyme source, and CSC was used as the substrate.
Fig. 11 is a graph showing a result of chromatography when PH20 was used as the enzyme source, and HA was used as the substrate.
Fig. 12 is a graph showing a result of chromatography when PH20 was used as the enzyme source, and CSA was used as the substrate.

### BEST MODE FOR CARRYING OUT THE INVENTION

The present invention is described below in detail based on the embodiments of the present invention.

### <1> Catalyst of the present invention

The catalyst of the present invention is a catalyst which comprises the following protein (A) or (B) as an essential component and is capable of specifically cleaving one sugar chain or two or more sugar chains selected from the group consisting of HA, CSA, CSC and CSD:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

The protein (A) is a protein of HYAL1.

In this connection, it is known that a mutation such as substitution, deletion, insertion, transposition or the like of amino acids can occur in the amino acid sequences of naturally distributing proteins due to a modification reaction or the like of the formed proteins inside the cells or during their purification, in addition to the polymorphism, mutation and the like of the DNA encoding the same, but in spite of this, some of them show physiological and biological activities which are substantially the same as those of the proteins having no mutation. Accordingly, a protein which has a structurally slight difference from the protein (A), but a great difference cannot be found regarding its function, can be used as an essential component of the catalyst of the present invention. The same is a case in which the above-described mutation is artificially introduced into the amino acid sequence of a protein, and in this case, it is possible to prepare a great variety of mutants. For example, it is known that a polypeptide in which a certain cysteine residue in the amino acid sequence of human interleukin 2 (IL-2) was substituted with serine keeps the interleukin 2 activity *(Science,* 224, 1431 (1984)). In addition, it is known that a certain kind of protein has a peptide region which is not essential for its activity. For example, the peptide region corresponds to a signal peptide existing in a protein to be secreted into the extracellular moiety and a pro sequence which can be found in a protease precursor or the like, and almost all of these regions are removed after their translation or in converting them into active type proteins. Such proteins are proteins which are present in the form of different primary structures but finally exert the same functions of the protein (A). The above-described protein (B) defines such proteins.

The term "several amino acids" as used herein represents the number of amino acids which can cause a mutation to such a degree that the activity of HYAL1 is not lost, and in the case of a protein consisting of 600 amino acid residues for example, it represents a number of approximately from 2 to 30, preferably from 2 to 15, more preferably from 2 to 8 or less. In the case of SEQ ID NO: 2, it represents a number of approximately from 2 to 22, preferably from 2 to 11, more preferably from 2 to 6 or less.

In the present specification, similar to the protein (B), a protein which consists of an amino acid sequence having a homology of 90% or more with the amino acid sequence represented by SEQ ID NO: 2, and has activity capable of specifically cleaving one sugar chain or two or more sugar chains selected from the group consisting of HA, CSA, CSC and CSD, can also be used as the active ingredient of the catalyst of the present invention.

It is preferable that the protein which consists of an amino acid sequence having a homology of 90% or more with the amino acid sequence represented by SEQ ID NO: 2, and has activity capable of specifically cleaving one sugar chain or two or more sugar chains selected from the group consisting of HA, CSA, CSC and CSD, has a homology of preferably 95% or more, more preferably 96% or more, and most preferably 97% or more, with the amino acid sequence represented by SEQ ID NO: 2. The homology of the amino acid sequence can be easily calculated by using conventionally known computer software such as FASTA, and such a software is also offered by Internet for use.

In addition, the amino acid sequences of the above-described proteins of (A) and (B) in the catalyst of the present invention may contain amino acid sequences of other protein and peptide. That is, the above-described proteins of (A) and (B) may be fusion proteins with other peptide. The present invention also provides such a fusion protein of the above-described protein (A) or (B) with other peptide (fusion protein of the present invention). The term "other peptide" in the present specification is used as a general idea including "polypeptide".

Examples of the fusion protein of the present invention include a fusion protein of HYAL1 with a marker peptide and the like. Such a fusion protein of the present invention has a merit of being able to facilitate purification and analysis. Examples of the marker peptide described in the above include FLAG peptide, protein A, insulin signal sequence, His, CBP (calmodulin-binding protein), GST (glutathione S-transferase) and the like. For example, a fusion protein with FLAG peptide can be conventionally purified by affinity chromatography using a solid phase to which an anti-FLAG antibody is bound. A fusion protein with protein A can be conventionally purified by affinity chromatography using a solid phase to which an IgG is bound. In the same manner, a solid phase to which magnetic nickel is bound can be used for a fusion protein with His tag. In addition, since a fusion protein with insulin signal is secreted into an extracellular moiety (medium, etc.), extraction operations such as cell disintegration become unnecessary. It is particularly preferable that the fusion protein of the present invention is a fusion protein with FLAG peptide.

The fusion protein of the present invention can be produced in the following manner.

Firstly, a DNA encoding the above-described protein (A) (a protein comprising an amino acid sequence represented by amino acid numbers 1 to 435 in SEQ ID NO: 2) (hereinafter referred to as "DNA (a)") is prepared. This DNA is not particularly limited, so long as it encodes a protein comprising an amino acid sequence represented by amino acid numbers 1 to 435 in SEQ ID NO: 2. Although various DNAs having different nucleotide sequences due to degeneracy of codons are present as such a DNA, a DNA identified by a nucleotide sequence represented by nucleotide numbers 1 to 1,308 in SEQ ID NO: 1 is preferable. This DNA is registered as GenBank Accession No. U03056.

In addition, a DNA encoding the above-described protein (B), instead of the above-described protein (A), (referred to as "DNA (b)") may also be used. The "DNA (b)" is not particularly limited, so long as it encodes a protein which comprises an amino acid sequence having deletion, substitution, insertion or transposition of one or several amino acids in the above-described amino acid sequence (A), and has activity capable of specifically cleaving one sugar chain or two or more sugar chains selected from the group consisting of HA, CSA, CSC and CSD.

Whether or not it has activity capable of cleaving these sugar chains can be detected by a general method for detecting sugar chains having a decreased molecular weight. For example, it can be detected based on the shift of sugar chain peaks by GPC which is shown later in Examples, and it is preferable to be detected by this method.

In addition, the presence or absence of specificity of activity capable of cleaving HA, CSA, CSC and CSD can be detected by detecting the presence or absence of the shift of sugar chain peaks by GPC using various substrates (sugar chains) other than HA, CSA, CSC and CSD. Deletion, substitution, insertion or transposition of amino acids keeping the activity capable of specifically cleaving one sugar chain or two or more sugar chains selected from the group consisting of HA, CSA, CSC and CSD can be easily selected by such a method.

Examples of such a "DNA (b)" include a DNA which hybridizes with the "DNA (a)", a DNA complementary to the DNA or a DNA having a nucleotide sequence of one of these DNA molecules, under stringent conditions. The "stringent conditions" as used herein are conditions under which so-called specific hybrid is formed but nonspecific hybrids are not formed (cf. Sambrook, J. *et al., Molecular Cloning, Laboratory Manual*, Second Edition, Cold Spring Harbor Laboratory Press (1989)). Specific examples of the "stringent conditions" include conditions in which a sample is hybridized at 42°C in a solution containing 50% formamide, 4 x SSC, 50 mM HEPES (pH 7.0), 10 x Denhardt's solution and 100 µg/ml salmon sperm DNA, and then washed with 2 x SSC and 0.1% SDS solution at room temperature and 0.1 × SSC and 0.1% SDS solution at 50°C.

It is preferable that the expression of a protein using a DNA comprising either one of the above-described DNA (a) and DNA (b) is carried out using a vector (preferably an expression vector) containing the DNA. The DNA can be inserted into a vector in the usual way.

Regarding the vector into which the DNA is inserted, for example, an appropriate expression vector (phage vector, plasmid vector, *etc.)* which can express the inserted DNA can be used, and it can be optionally selected according to the host cell into which the vector of the present invention is introduced. Examples of such a host-vector system include a combination of *Escherichia coli (E. coli)* with an expression vector for procaryotic cell such as pET15b (manufactured by Novagen), pTrcHis (manufactured by Invitrogen), pGEX (manufactured by Pharmacia Biotech), pTrc99 (manufactured by Pharmacia Biotech), pKK233-3 (manufactured by Pharmacia Biotech), pEZZZ18 (manufactured by Pharmacia Biotech), pCH110 (manufactured by Pharmacia Biotech), pBAD (manufactured by Invitrogen), pRSET (manufactured by Invitrogen) or pSE420 (manufactured by Invitrogen), and a combination of a mammalian cell such as COS cell or 3LL-HK46 cell with an expression vector for mammalian cell such as pGIR201 (Kitagawa, H. and Paulson, J.C., *J. Biol. Chem*. 269, 1394-1401 (1994)), pEF-BOS (Mizushima, S. and Nagata, S., *Nucleic Acid Res.,* 18, 5322 (1990)), pCXN2 (Niwa, H., Yamanura, K. and Miyazaki, J., *Gene*, 108, 193-200 (1991)), pCMV-2 (manufactured by Eastman Kodak), pCEV18, pME18S (Maruyama *et al*., *Med. Immunol.,* 20, 27 (1990)) or pSVL (manufactured by Pharmacia Biotech), as well as yeast, *Bacillus subtilis* and the like as host cells and various vectors corresponding to them. Among the above-described host-vector systems, a combination of a prokaryotic cell (particularly *E. coli* cell) with pET15b is particularly preferred.

In addition, an insect cell can also be used as the host cell. Also, in this case, various vectors corresponding to respective insect cells can be used. As the insect cell, it is preferable to use a high expression SF+ cell (expresSf+ (registered trademark)). In addition, pPSC8 or pPSC12 is preferable as a transfer vector into which the DNA is inserted. When the DNA is inserted into pPSC8, its insertion can be carried out using *Sma*I, *Kpn*I, *Pst*I, *Xba*I, *Nru*I and *Bgl*II. When the DNA is inserted into pPSC12, its insertion can be carried out using *Sma*I, *Kpn*I, *PstI* or *Bgl*II. Also, in order to express a protein in such a manner that FLAG peptide is fused to its N-terminus, it is preferable to add a sequence encoding the FLAG peptide to a terminus of the corresponding DNA, prior to integrating into the transfer vector.

After the DNA is inserted into the transfer vector, it is preferable to introduce this vector to a baculovirus. As the baculovirus, it is preferable to use *Autographa californica* nucleopolyhedrovirus (AcNPV) or *Bombyx mori* nucleopolyhedrovirus (BmNPV). Thereafter, an insect cell is infected with the baculovirus introduced with the DNA of interest.

Regarding the vector into which a DNA is inserted, those which are constructed in such a manner that they can express a fusion protein of the protein of interest with a marker peptide can be used. Expression of the protein from DNA and collection of the expressed protein can also be carried out in accordance with general methods.

For example, they can be carried out by introducing, into an appropriate host, an expression vector inserted with the DNA of interest to thereby transform the host, growing the transformant, and then collecting the expressed protein from the grown matter.

The catalyst of the present invention is capable of specifically cleaving one sugar chain or two or more sugar chains selected from the group consisting of HA, CSA, CSC and CSD, and the presence or absence of this capability can also be detected by a general detection method of sugar chains having a decreased molecular weight. The method is similar to that described above. A catalyst which is capable of specifically cleaving 4 kinds of sugar chains, HA, CSA, CSC and CSD is particularly preferred.

Also, it is preferable that the catalyst of the present invention is not capable of cleaving DS, KS, and CH having an average molecular weight of 7,000. Whether or not the catalyst is capable of cleaving DS, KS, and CH having an average molecular weight of 7,000 can also be detected by a general detection method of sugar chains having a decreased molecular weight by using, as the substrate, DS, KS, or CH having an average molecular weight of 7,000 as described above.

The catalyst of the present invention comprises at least the above-described protein (A) or (B). That is, the catalyst of the present invention may use the protein itself of the above-described (A) or (B) as the catalyst of the present invention, or may contain another component in addition to the above-described protein (A) or (B), so long as it does not has bad influence upon these proteins and also does not has influence upon the effect of the present invention.

The method for producing the catalyst of the present invention is not particularly limited, and the above-described protein (A) or (B) may be isolated from a natural material, the above-described protein (A) or (B) may be produced by chemical synthesis or the like, or the above-described protein (A) or (B) may be produced by a genetic engineering means. The method for producing the catalyst of the present invention by a genetic engineering means should be referred to the method for producing the catalyst of the present invention which is described later.

Form of the catalyst of the present invention is not limited, and it may be any one of a liquid form, a frozen form, a freeze-dried form and an immobilized enzyme form in which it is conjugated to a carrier.

### <2> Cleavage agent of the present invention

The cleavage agent of the present invention is an agent which comprises the following protein (A) or (B) as an essential component and is specific for cleavage of one sugar chain or two or more sugar chains selected from the group consisting of HA, CSA, CSC and CSD:
(A) a protein which comprises the amino acid sequence represented by SEQ ID NO:2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

The cleavage agent of the present invention has activity capable of specifically cleaving one sugar chain or two or more sugar chains selected from the group consisting of HA, CSA, CSC and CSD, and the presence or absence of this capability can also be detected by a general detection method of sugar chains having a decreased molecular weight. The method is similar to that described above. It is particularly preferable that the cleavage agent is capable specifically cleaving 4 kinds of sugar chains, HA, CSA, CSC and CSD.

Also, it is preferable that the cleavage agent of the present invention is not capable of cleaving DS, KS, and CH having an average molecular weight of 7,000. The presence or absence of this capability of cleaving DS, KS, and CH having an average molecular weight of 7,000 can also be detected by a general detection method of sugar chains having a decreased molecular weight by using, as the substrate, DS, KS, or CH having an average molecular weight of 7,000 as described above.

Although the cleavage agent of the present invention is used for specifically cleaving one sugar chain or two or more sugar chains selected from the group consisting of HA, CSA, CSC and CSD, it is preferable to use the cleavage agent for the purpose of not cleaving DS, KS, and CH having an average molecular weight of 7,000 under such an obj ect.

Conventionally known methods can be used for making the cleavage agent of the present invention into a pharmaceutical preparation. In addition, the form of the cleavage agent of the present invention is not limited, and it may be any one of a liquid form, a frozen form, a freeze-dried form and an immobilized enzyme form in which it is conjugated to a carrier.

Other descriptions regarding the cleavage agent of the present invention are the same as the above-described "<1> Catalyst of the present invention". The method for producing the cleavage agent of the present invention by a genetic engineering means should be referred to the method for producing the cleavage agent of the present invention which is described later.

### <3> Medicament of the present invention

The medicament of the present invention is a medicament which comprises the following protein (A) or (B) as an essential component and is used for specifically cleaving one sugar chain or two or more sugar chains selected from the group consisting of HA, CSA, CSC and CSD which are present in a living body tissue:
(A) a protein which comprises the amino acid sequence represented by SEQ ID NO:2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

The medicament of the present invention has activity capable of specifically cleaving one sugar chain or two or more sugar chains selected from the group consisting of HA, CSA, CSC and CSD, and the presence or absence of this capability can also be detected by a general detection method of sugar chains having a decreased molecular weight. The method is similar to that described above. It is particularly preferable that the medicament is capable of specifically cleaving 4 kinds of sugar chains, HA, CSA, CSC and CSD.

Also, it is preferable that the medicament of the present invention is not capable of cleaving DS, KS, and CH having an average molecular weight of 7,000. The presence or absence of this capability of cleaving DS, KS, and CH having an average molecular weight of 7,000 can also be detected by a general detection method of sugar chains having a decreased molecular weight by using, as the substrate, DS, KS, or CH having an average molecular weight of 7,000 as described above.

Although the medicament of the present invention is used for specifically cleaving one sugar chain or two or more sugar chains selected from the group consisting of HA, CSA, CSC and CSD, it is preferable to use the medicament for the purpose of not cleaving DS, KS, and CH having an average molecular weight of 7,000 under such an object.

The living body tissue to which the medicament of the present invention is applied is not particularly limited, so long as it is a living body tissue which is required for specific cleavage of one sugar chain or two or more sugar chains selected from the group consisting of HA, CSA, CSC and CSD. An example thereof is a living body tissue in which one sugar chain or two or more sugar chains selected from the group consisting of HA, CSA, CSC and CSD are present excessively at a level higher than that under the normal conditions. Such a living body tissue specifically includes nucleus pulposus, and a nucleus pulposus under conditions of disc herniation is particularly preferable. That is, it is preferable that the medicament of the present invention is an agent for treating disc herniation. The term "treatment" as used in this specification includes treatments for prevention, inhibition of advance (prevention of worsening), improvement, cure and the like.

) In recent years, attempts have actually been made to treat disc herniation by degrading nucleus pulposus through the administration of chondroitinase into an intervertebral disc (US Patent No. 4,696,816, *Clinical Orthopaedics,* 253, 301-308 (1990)). Based on these attempts, the medicament of the present invention having activity capable of cleaving CS can also be used sufficiently as a treating agent for disc herniation.

In addition, the animal to which the medicament of the present invention is applied is not particularly limited, but a vertebrate is preferable, and a mammal is particularly preferable. Examples of the mammal include human, dog, cat, rabbit, horse, sheep and the like, but a medicament which can be applied to human is particularly preferable.

The administration method of the medicament of the present invention is not particularly limited, so long as the medicament of the present invention has activity capable of specifically cleaving one sugar chain or two or more sugar chains selected from the group consisting of HA, CSA, CSC and CSD in a living body tissue, and examples include administration by injection. When the medicament of the present invention is applied to a nucleus pulposus, it is preferable to inject it into an intervertebral disc or vertebral epidural space where the nucleus pulposus of interest is present.

The protein of the above-described (A) or (B) can be made into the medicament of the present invention by optionally making it into a pharmaceutical preparation according to the administration method. In general, the active ingredient is mixed with one or more of pharmaceutically acceptable carriers and made into pharmaceutical preparations by optional methods well known in the technical field of pharmaceutics. The dosage forms include injections (solution, suspension, emulsion, solid preparation for dissolution before use, *etc*.), tablets, capsules, solutions and the like. Among these, injections are preferable.

The dose of the medicament of the present invention is not particularly limited, because it should be individually set based on the kinds and specific activity of the above-described protein (A) or (B), kinds, symptoms and the like of the animal to be administered, and kinds, conditions and the like of the living body tissue as the object of administration, but in general, approximately from 0.1 µg to 1,000 mg per day can be administered.

Other descriptions regarding the medicament of the present invention are the same as the above-described "<1> Catalyst of the present invention". The method for producing the medicament of the present invention by a genetic engineering means should be referred to the method for producing the medicament of the present invention which is described later.

### <4> Cleavage method of the present invention

The cleavage method of the present invention is a method for specifically cleaving one sugar chain or two or more sugar chains selected from the group consisting of HA, CSA, CSC and CSD, which comprises at least the step reacting the following protein (A) or (B) with the sugar chain(s):
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

Descriptions on the above-described proteins (A) and (B) are the same as in the above-described "<1> Catalyst of the present invention". The method for reacting the above-described protein (A) or (B) with one sugar chain or two or more sugar chains selected from the group consisting of HA, CSA, CSC and CSD is not particularly limited, so long as these molecules contact with each other and become a state of occurring the enzyme reaction, so that, for example, the latter may be added to the former, the former may be added to the latter, or both may be simultaneously added.

Alternatively, the above-described proteins of (A) and (B) may be immobilized on a carrier (e.g., gel, bead, membrane, plate, *etc*.) and react with one sugar chain or two or more sugar chains selected from the group consisting of HA, CSA, CSC and CSD.

Conditions of the reaction after allowing both of them to react with each other are not particularly limited, so long as they are conditions under which the above-described protein (A) or (B) can act, but it is preferable to carry out the reaction at around the optimum pH of these proteins (e.g., approximately from pH 4 to pH 5), and it is more preferable to carry out the reaction in a buffer solution having a buffer action at the pH. Also, the temperature in this case is not particularly limited, so long as the activities of these proteins are maintained, and a temperature of approximately from 35°C to 40°C can be exemplified. In addition, when a substance which increases the activities of these proteins is available, this substance may be added. The reaction time can be optionally adjusted according to the pH conditions, temperature conditions, amounts of the protein and sugar chain to be reacted, desired degree of cleavage (molecular weight reduction) and the like. The degree of cleavage (molecular weight reduction) can be increased by prolonging the reaction time, and the degree can be decreased by shortening the reaction time.

Also, the cleavage method of the present invention comprises at least such an action step of protein, and it may further contain other steps.

By the cleavage method of the present invention, one sugar chain or two or more sugar chains selected from the group consisting of HA, CSA, CSC and CSD are specifically cleaved. Whether or not these sugar chains have been cleaved can be detected by a general detection method of sugar chains having a decreased molecular weight. The method is similar to that described above. A method for specifically cleaving 4 kinds of sugar chains, HA, CSA, CSC and CSD is particularly preferred.

In addition, it is preferable that the cleavage method of the present invention does not cleave DS, KS, and CH having an average molecular weight of 7,000. The absence of cleaving of DS, KS, and CH having an average molecular weight of 7,000 can also be detected by a general detection method of sugar chains having a decreased molecular weight.

That is, the cleavage method of the present invention is used for specifically cleaving one sugar chain or two or more sugar chains selected from the group consisting of HA, CSA, CSC and CSD, but it is preferable to use this method for the purpose of not cleaving DS, KS, and CH having an average molecular weight of 7,000 under such an object.

Other descriptions regarding the cleavage method of the present invention are the same as in the above-described "<1> Catalyst of the present invention".

### <5> Production method of the sugar chain of the present invention

The production method of the sugar chain of the present invention is a method for specifically producing a sugar chain(s) having a decreased molecular weight, which comprises reacting the following protein (A) or (B) with one sugar chain or two or more sugar chains selected from the group consisting of HA, CSA, CSC and CSD:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

By the production method of the sugar chain of the present invention, sugar chains in which molecular weights of one sugar chain or two or more sugar chains selected from the group consisting of HA, CSA, CSC and CSD are decreased can be specifically produced. Whether or not the sugar chains having a decreased molecular weight have been produced can be detected by a general detection method of sugar chains having a decreased molecular weight. The method is similar to that described above. It is particularly preferable that the method is a method for specifically producing 4 kinds of sugar chains, HA, CSA, CSC and CSD.

Also, it is preferable that the production method of the sugar chain of the present invention does not produce DS, KS and CH having a decreased molecular weight. The absence of the production of DS, KS and CH can also be confirmed by a general detection method of sugar chains having a decreased molecular weight.

That is, the production method of the sugar chain of the present invention is used for specifically cleaving one sugar chain or two or more sugar chains selected from the group consisting of HA, CSA, CSC and CSD, and it is preferable to use this method for the purpose of not cleaving DS, KS, and CH having an average molecular weight of 7,000 under such an object.

The method for isolating sugar chains having a decreased molecular weight from the product, and the like, can be carried out by conventionally known methods.

Other descriptions regarding the production method of the sugar chain of the present invention are the same as in the above-described "<1> Catalyst of the present invention" and "<4> Cleavage method of the present invention".

### <6> Production method of the catalyst of the present invention

The production method of the catalyst of the present invention is a method for producing the catalyst of the present invention, which comprises expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

The above-described DNA of (a) is not particularly limited, so long as it encodes a protein consisting of the amino acid sequence represented by SEQ ID NO: 2. Although various DNAs having different nucleotide sequences due to degeneracy of codons are present as such a DNA, a DNA specified by the nucleotide sequence represented by SEQ ID NO: 1 is preferable. The DNA represented by SEQ ID NO: 1 is registered as GenBank Accession No. U03056.

The above-described DNA of (b) is not particularly limited, so long as it encodes a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD. Examples of such a DNA include a DNA which hybridizes with the DNA described in the above-described (a) or a DNA complementary to the DNA or a DNA having a nucleotide sequence of one of these DNA molecules, under stringent conditions.

The "stringent conditions" as used herein are conditions under which so-called specific hybrid is formed but nonspecific hybrids are not formed (cf. Sambrook, J. *et al., Molecular Cloning, A Laboratory Manual*, Second Edition, Cold Spring Harbor Laboratory Press (1989), *etc.).* A specific example of the "stringent conditions" are conditions in which a sample is hybridized at 42°C in a solution containing 50% formamide, 4 x SSC, 50 mM HEPES (pH 7.0), 10 x Denhardt's solution and 100 µg/ml salmon sperm DNA, and then washed with 2 x SSC and 0.1% SDS solution at room temperature and then with 0.1 x SSC and 0.1% SDS solution at 50°C.

It is preferable that the expression of a protein using a DNA comprising either one of the above-described DNA (a) and DNA (b) is performed using a vector (preferably an expression vector) containing the DNA. The DNA can be inserted into a vector in the usual way.

Regarding the vector into which the DNA is inserted, for example, an appropriate expression vector (a phage vector, plasmid vector, *etc*.) which can express the inserted DNA can be used, and it can be optionally selected according to the host cell into which the vector of the present invention is introduced. Examples of such a host-vector system include a combination of a mammalian cell such as COS cell or 3LL-HK46 cell with an expression vector for mammalian cell such as pGIR201 (Kitagawa, H. and Paulson, J.C., *J. Biol. Chem.* 269, 1394-1401 (1994)), pEF-BOS (Mizushima, S. and Nagata, S., *Nucleic Acid Res*. 18, 5322 (1990)), pCXN2 (Niwa, H., Yamanura, K. and Miyazaki, J., *Gene,* 108, 193-200 (1991)), pCMV-2 (manufactured by Eastman Kodak), pCEV18, pME18S (Maruyama *et al*., *Med*. *Immunol.,* 20, 27 (1990)) or pSVL (manufactured by Pharmacia Biotech), a combination of *Escherichia coli (E. coli)* with an expression vector for procaryotic cell such as pTrcHis (manufactured by Invitrogen), pGEX (manufactured by Pharmacia Biotech), pTrc99 (manufactured by Pharmacia Biotech), pKK233-3 (manufactured by Pharmacia Biotech), pEZZZ18 (manufactured by Pharmacia Biotech), pCH110 (manufactured by Pharmacia Biotech), pET (manufactured by Stratagene), pBAD (manufactured by Invitrogen), pRSET (manufactured by Invitrogen) or pSE420 (manufactured by Invitrogen), and a combination of an insect cell with a baculovirus, as well as yeast, *Bacillus subtilis* and the like as host cells and various vectors corresponding to them. Among the above-described host-vector systems, a combination of a mammalian cell (particularly COS cell) with pFLAG-CMV6 (manufactured by SIGMA) is particularly preferable.

In addition, regarding the vector into which a DNA is inserted, those which are constructed in such a manner that a fusion protein of the protein of interest with a marker peptide can be expressed. Expression of a protein from DNA and collection of the expressed protein can also be carried out in accordance with general methods.

For example, they can be carried out by introducing, into an appropriate host, an expression vector inserted with the DNA of interest to transform the host, growing this transformant, and then collecting the expressed protein from the grown matter.

The term "growing" as used herein is a general idea including growth of a cell or a microorganism itself as the transformant and growth of an animal, insect or the like integrated with a cell as the transformant. Also, the term "grown matter" as used herein is a general idea including a medium (supernatant of cultured medium) after growth of a transformant and cultured host cell, secreted product, excreted product and the like. Growth conditions (medium, culture conditions, *etc*.) can be optionally selected according to the host to be used.

A protein from the grown matter can also be collected by conventionally known extraction and purification methods of protein.

For example, when the protein of interest is produced in a soluble form secreted into a medium (supernatant of cultured medium), the medium may be collected and used as such. In addition, when the protein of interest is produced in a soluble form secreted into cytoplasm or in an insoluble form (membrane-binding), the protein of interest can be extracted by cell disruption extraction using a method which uses a nitrogen cavitation device, a homogenizer, a glass beads mill method, an ultrasonic wave treatment, an osmotic shock method, a freeze-thawing method or the like, extraction with a surfactant, or a combination thereof, and the extract may be used as such.

The protein can be further purified from these media or extracts. The purification may be an imperfect purification (partial purification) or complete purification, which can be optionally selected according to the using purpose and the like of the protein of interest.

Examples of the purification method include salting out by ammonium sulfate, sodium sulfate or the like, centrifugation, dialysis, ultrafiltration, adsorption chromatography, ion exchange chromatography, hydrophobic chromatography, reverse phase chromatography, gel filtration, gel permeation chromatography, affinity chromatography, electrophoresis, a combination thereof and the like.

Whether or not the protein of interest was produced can be confirmed by analyzing its amino acid sequence, action, substrate specificity and the like.

In addition, the cleavage method of the present invention comprises at least the expression and collection steps described in the above, and may further comprise other steps.

### <7> Cleavage agent producing method of the present invention

The cleavage agent producing method of the present invention is a method for producing the cleavage agent of the present invention, which comprises at least the step expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

Other descriptions on the cleavage agent producing method of the present invention are the same as in the above-described "<6> Production method of the catalyst of the present invention".

### <8> Medicament producing method of the present invention

The medicament producing method of the present invention is a method for producing the medicament of the present invention, which comprises at least the step expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of HA, CSA, CSC and CSD.

Other descriptions on the medicament producing method of the present invention are the same as in the above-described "<6> Production method of the catalyst of the present invention".

### <9> Treating method of the present invention

The treating method of the present invention is a method for treating a disease in which one sugar chain or two or more sugar chains selected from the group consisting of HA, CSA, CSC and CSD is/are excessively present in a living body tissue, which comprises administering the medicament of the present invention.

Other descriptions on the treating method of the present invention are the same as in the above-described "<3> Medicament of the present invention".

Examples of the present invention are specifically described in the following. However, the technical scope of the present invention is not limited thereto.

### (1) Expression of HYAL1 gene

COS-7 cells (obtained from Bio Resource Center, RIKEN) were cultured in DMEM medium (manufactured by Invitrogen) containing 10% fetal bovine serum until they reached about 20 to 40% confluent. The culturing was carried out at 37°C under 5% CO₂.

A hyaluronidase expression vector prepared by inserting the nucleotide sequence represented by SEQ ID NO: 1 (cDNA of HYAL1) into a pFLAG-CMV6 vector (manufactured by SIGMA-Aldrich) was transfected into the COS-7 cells using TransFast Transfection kit (manufactured by Promega), and the cells were cultured at 37°C for 3 days in the DMEM medium containing 10% fetal bovine serum. The culturing was carried out at 37°C under 5% CO₂.

In addition, cells transfected with the pFLAG-CMV6 vector into which the nucleotide sequence represented by SEQ ID NO: 1 (cDNA of HYAL1) was not inserted were also cultured in the same manner.

### (2) Preparation of enzyme solution

The cells cultured in the above-described (1) (those which were transfected with the expression vector into which the cDNA of HYAL1 had been inserted, or transfected with the expression vector into which the cDNA of HYAL1 had not been inserted) were disrupted under ice-cooling using a cell scraper in a solution containing 10 mM Tris-HCl (pH 7.4 or pH 6.0), 0.5% Triton X-100 and 0.25 M sucrose. This liquid after disruption was directly used as an enzyme solution.

### (3) Analysis of substrate specificity

Substrate specificity of the enzyme was analyzed by allowing the enzyme solution prepared in (2) to react with various substrates. The reaction was carried out by adding the enzyme solution prepared in (2) to a reaction mixture containing 0.25% of respective substrate, 0.15 M NaCl, and 50 mM sodium acetate buffer (pH 4 or pH 5) or sodium phosphate buffer (pH 6 or pH 7), to a final concentration of 20% (vol/vol), and incubating the mixture at 37°C for 4 days.

The substrates used herein are as follows.
* HS: (derived from chicken crest; manufactured by Seikagaku Corporation)
* CH: (derived from shark; manufactured by Seikagaku Corporation), average molecular weight of 7,000
* CSA (derived from whale cartilage; manufactured by Seikagaku Corporation)
   This CS is available from Seikagaku Corporation as a reagent catalogue Code No. 400650 of the same company, and has the following properties:
   showing a single band by cellulose acetate membrane electrophoresis;
   nitrogen content: 2.4 to 2.8% (measured by the method described in *Z*. *Anal. Chem.,* 22, p. 366 (1883));
   sulfur content: 6.2 to 6.6% (measured by the method described in *Mikrochim. Acta.,* 123 (1955));
   galactosamine content: 32.0 to 37.0% (automatic amino acid analyzer);
   glucuronic acid content: 35.0 to 40.0% (carbazole reaction);
   molecular weight: 25,000 to 50,000 (measured by the method described in
   *Biochem. J*., 23, p. 517 (1929));
   4-sulfate/6-sulfate: 80/20 (measured by the method described in *J. Biol. Chem.,* 243, p. 1536 (1968)).
* DS (derived from swine skin; manufactured by Seikagaku Corporation)
   This DS (CSB) is available from Seikagaku Corporation as a reagent catalogue Code No. 400660 of the same company, and has the following properties:
   judged pure by infrared spectrophotometer;
   nitrogen content: 2.4 to 2.9% (measured by the method described in Z. *Anal. Chem.,* 22, p. 366 (1883));
   sulfur content: 6.2 to 6.9% (measured by the method described in *Mikrochim. Acta.,* 123 (1955));
   galactosamine content: 32.0 to 37.0% (automatic amino acid analyzer);
   iduronic acid content: 36.0 to 42.0% (carbazole reaction);
   molecular weight: 11,000 to 25,000 (measured by the method described in *Biochem. J*., 23, p. 517 (1929)).
* CSC (derived from shark cartilage; manufactured by Seikagaku Corporation)
   This CS is available from Seikagaku Corporation as a reagent catalogue Code No. 400670 of the same company, and has the following properties:
   showing a single band by cellulose acetate membrane electrophoresis;
   nitrogen content: 2.3 to 2.7% (measured by the method described in Z. *Anal. Chem.,* 22, p. 366 (1883));
   sulfur content: 6.4 to 6.8 (measured by the method described in *Mikrochim. Acta.,* 123 (1955));
   galactosamine content: 32.0 to 37.0% (automatic amino acid analyzer);
   glucuronic acid content: 34.0 to 39.0% (carbazole reaction);
   molecular weight: 40,000 to 80,000 (measured by the method described in *Biochem. J*., 23, pp 517 (1929));
   4-sulfate/6-sulfate: 10/90 (measured by the method described in *J. Biol. Chem.,* 243, p. 1536 (1968))
* CSD (derived from shark cartilage; manufactured by Seikagaku Corporation)
   This CS is available from Seikagaku Corporation as a reagent catalogue Code No. 400676 of the same company, and has the following properties:
   nitrogen content: 2.2 to 2.6% (measured by the method described in *Z. Anal. Chem.,* 22, p. 366 (1883));
   sulfur content: 7.1 to 7.7% (measured by the method described in *Mikrochim. Acta.,* 123 (1955));
   galactosamine content: 30 to 35% (automatic amino acid analyzer);
   glucuronic acid content: 32 to 35% (carbazole reaction)
* KS (derived from bovine cornea; manufactured by Seikagaku Corporation)
   This KS is available from Seikagaku Corporation as a reagent catalogue Code No. 400760 of the same company, and has the following properties:
   showing a single band by cellulose acetate membrane electrophoresis;
   nitrogen content: 2.2 to 3.0% (measured by the method described in *Z. Anal. Chem.,* 22, p. 366 (1883));
   sulfur content: 6.5 to 8.0% (measured by the method described in *Mikrochim. Acta.,* p. 123 (1955));
   galactose content: 34 to 40% (measured by the method described in *Biochem. J.,* 50, p. 298 (1952));
   galactosamine content: lower than the detection limit (automatic amino acid analyzer);
   glucosamine content: 30 to 40% (HITACHI KLA-5; manufactured by Hitachi)

The solution after incubation was subjected to gel permeation chromatography (column: three columns consists of TSKgel-G2500PWXL, TSKgel-G3000PWXL and TSKgel-G4000PWXL, eluent was 0.2 M NaCl, flow rate was 0.6 ml/min) to observe shifting of the position of refraction index.

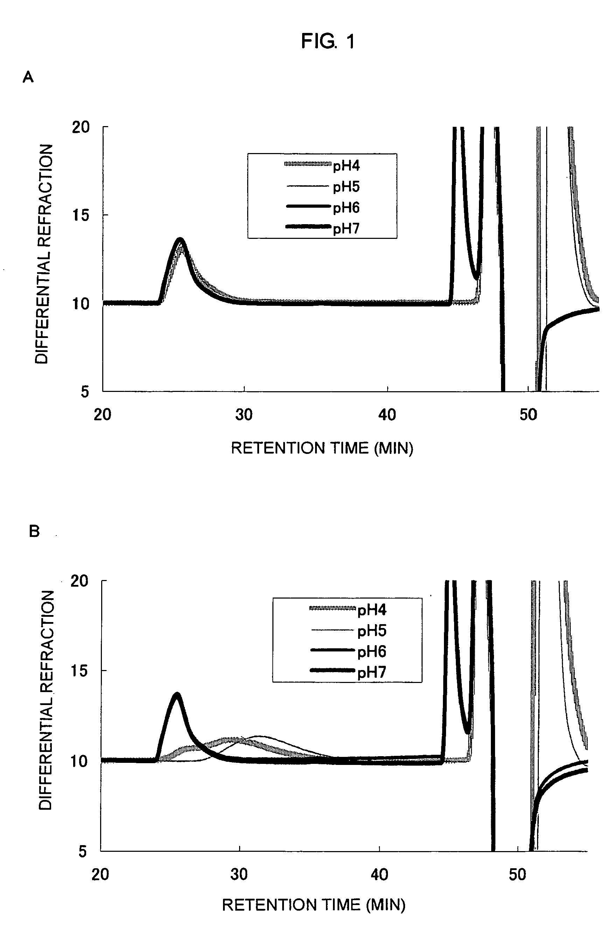

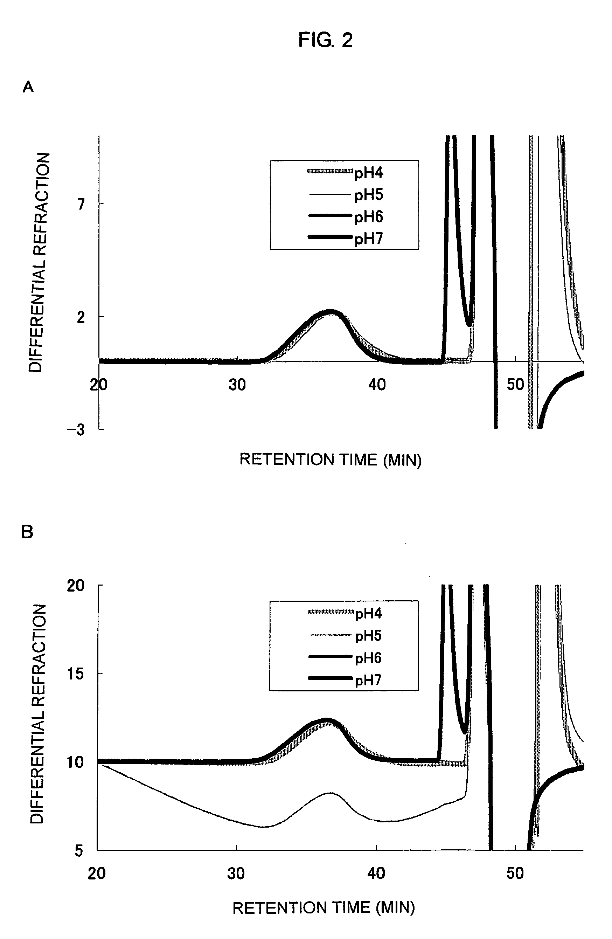

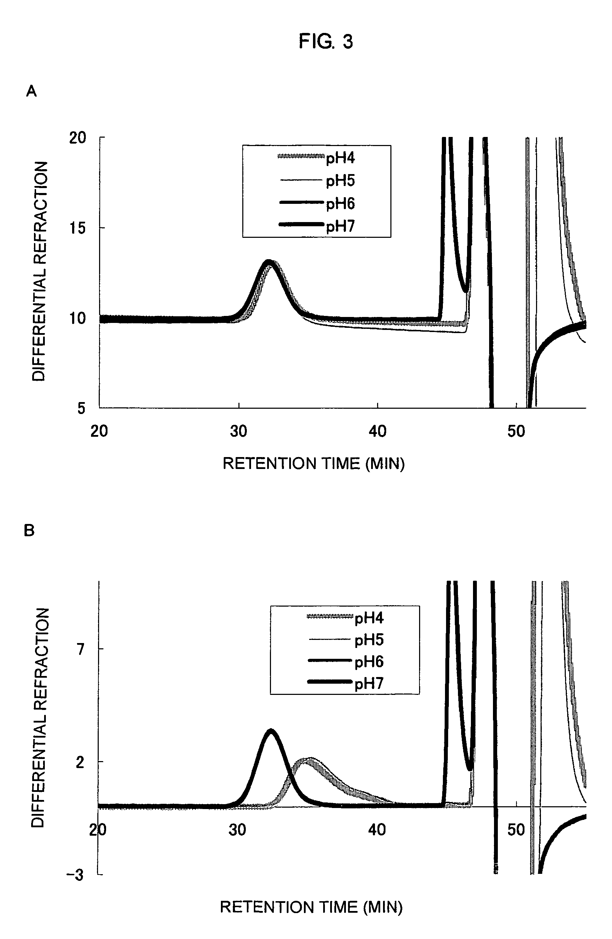

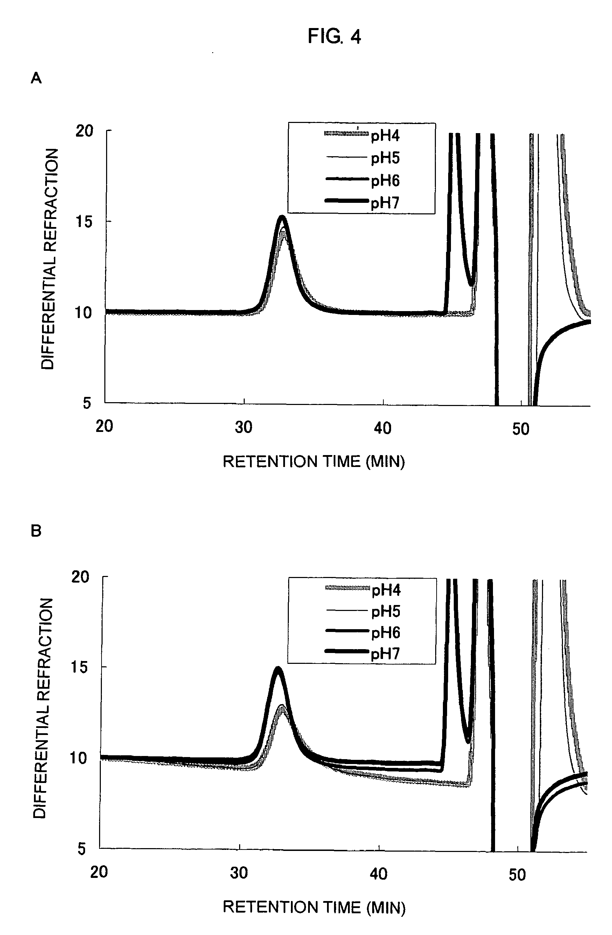

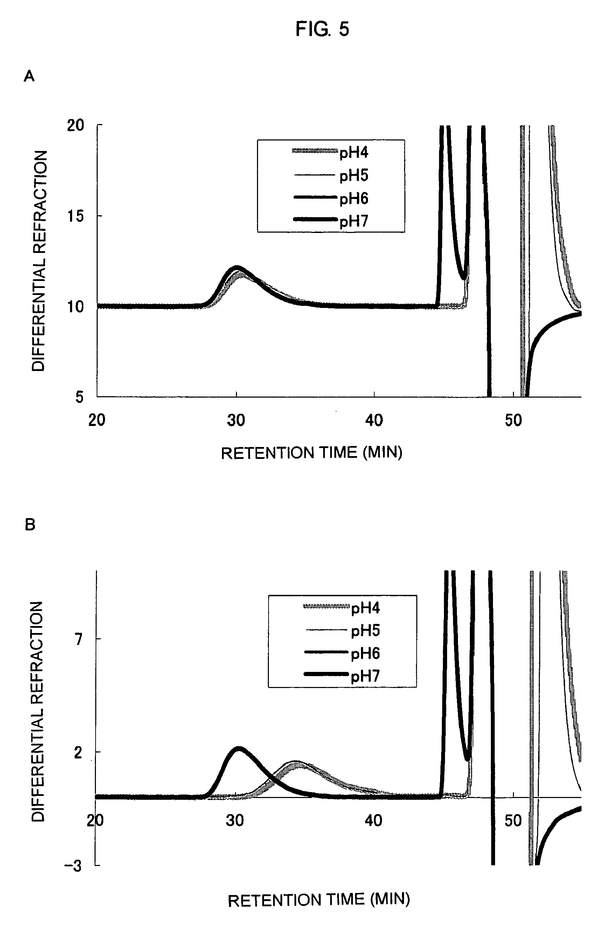

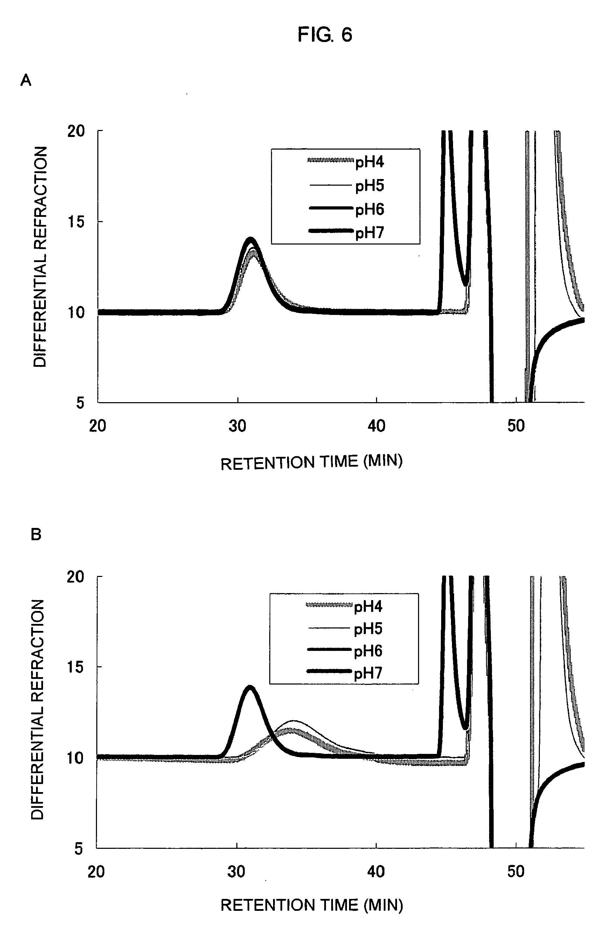

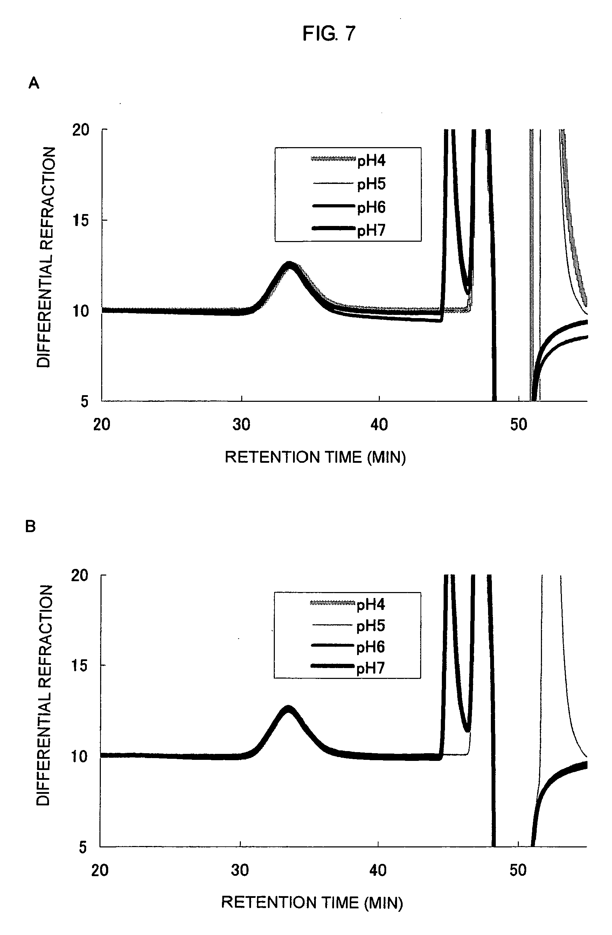

A result when HA was used as the substrate is shown in Fig. 1, and a result when CH having an average molecular weight of 7,000 was used as the substrate is shown in Fig. 2, a result when CSA was used is shown in Fig. 3, a result when CSB was used is shown in Fig. 4, a result when CSC was used is shown in Fig. 5, a result when CSD was used is shown in Fig. 6, and a result when KS was used is shown in Fig. 7. The vertical axis of each graph shows differential refraction, and the horizontal axis shows time (retention time; min). The upper side of each drawing ("A" in each drawing) is a result when the enzyme solution of cells not transfected with the cDNA of HYAL1 was used, and the lower side of each drawing ("B" in each drawing) is a result when the enzyme solution of cells transfected with the cDNA of HYAL1 was used. Also, the thickest solid line (blue line) in the drawing shows a result when the reaction was carried out in a reaction mixture of pH 7, and the second thick solid line (red line) shows that of pH 6, the thinnest solid line (green line) shows that of pH 5, and the dotted line (black line) shows that of pH 4. When the retention time of the peak locating at about the center of each graph is shifted to a longer time side than the graph of the upper side (not transfected with the cDNA of HYAL1), it means that molecular weight of the substrate was decreased.

As shown in Fig. 1, the peak locating at about the center shifted to the longer time side when the enzyme solution of cells transfected with the cDNA of HYAL1 was allowed to react with HA under conditions of pH 4 or pH 5 (molecular weight of HA was decreased). Based on this result, it was confirmed that HYAL1 having activity capable of cleaving HA is actually expressed in cells transfected with the cDNA of HYAL1.

As shown in Fig. 2, Fig. 4 and Fig. 7, shifting of the peak locating at about the center was not observed when the enzyme solution of cells transfected with the cDNA of HYAL1 was allowed to react with CSB, KS, or CH having an average molecular weight of 7,000 under conditions of pH 4 to 7 (molecular weight of these substrates was not decreased). Based on this result, it was shown that HYAL1 does not substantially have activity capable of cleaving CSB, KS, and CH having an average molecular weight of 7,000.

As shown in Fig. 3, Fig. 5 and Fig. 6, the peak locating at about the center shifted to the longer time side when the enzyme solution of cells transfected with the cDNA of HYAL1 was allowed to react with CSA, CSC or CSD under conditions of pH 4 or pH 5 (molecular weight of these substrates was decreased). Based on this result, it was shown that HYAL1 has activity capable of cleaving CSA, CSC and CSD.

Based on the above results, it was shown that HYAL1 has activity capable of cleaving not only HA but also CSA, CSC and CSD, but does not substantially have activity capable of cleaving DS (CSB), KS, and CH having an average molecular weight of 7,000.

In addition, since the solution after incubation did not show the absorption at 210 nm which reflects unsaturated saccharides, it was shown that HYAL1 cleaves CSA, CSC and CSD by hydrolysis.

### (4) Analysis of reactivity with substrates

The reactivity of HYAL1 with substrates was compared with the reactivity of PH20 (testis hyaluronidase: hyaluronidase registered as GenBank Accession No. S67798) with substrates.

The enzyme solution and substrates used herein are the same as the enzyme solution and substrates used in (3). In addition, the composition of the reaction solution and the reaction temperature are also the same as in (3). However, the reaction time was set for 5 points of 1 day, 4 days, 7 days, 14 days and un-reaction, and each of the samples after completion of the reaction was cryopreserved at -30°C.

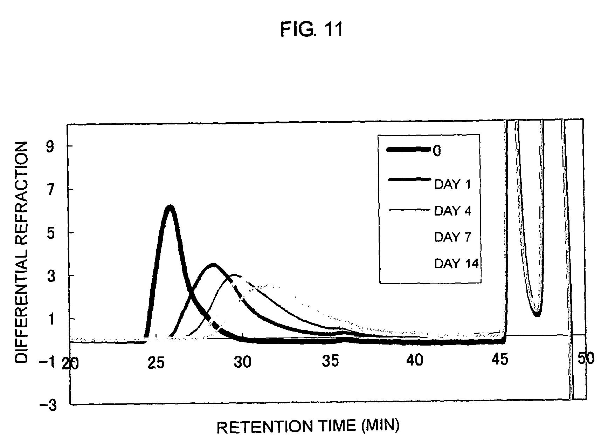

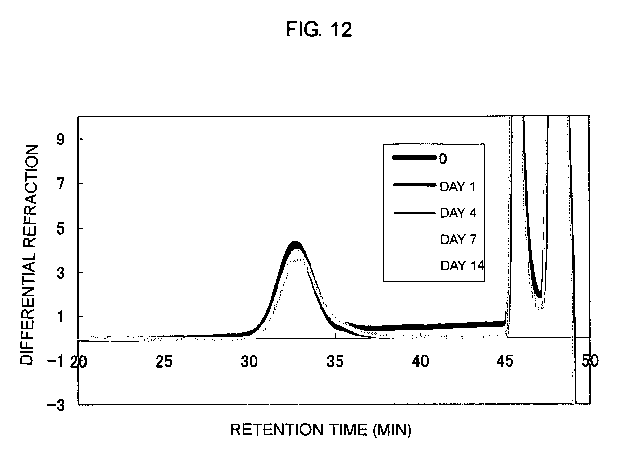

Also, the enzyme solution of PH20 used in Fig. 11 and Fig. 12 was prepared in the same manner as the case of HYAL1 by the method described in (1) and (2). The cDNA of PH20 was inserted into pFLAG-CMV6 vector, and the hyaluronidase expression vector was introduced into COS cells under the same conditions of (1) for transient expression of the PH20 protein. The expressed cells were disrupted under the same conditions as (2) and used as the enzyme solution.

Shifting of the position of differential refraction peak on each sample after the reaction was observed by using gel permeation chromatography in the same manner as in (3).

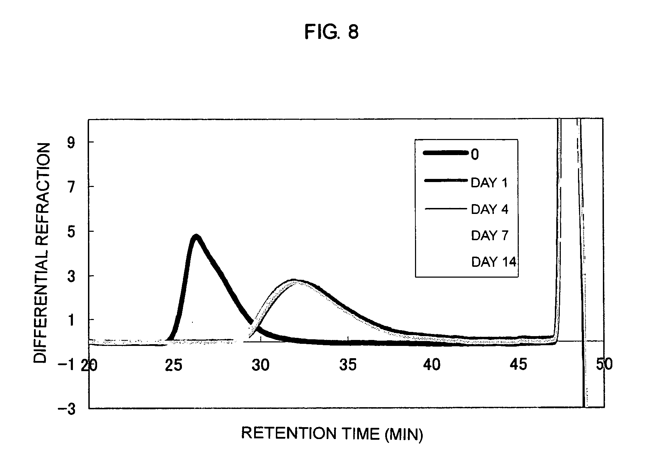

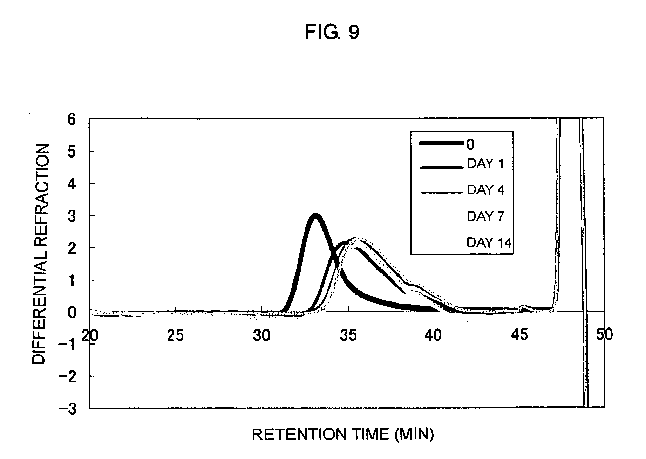

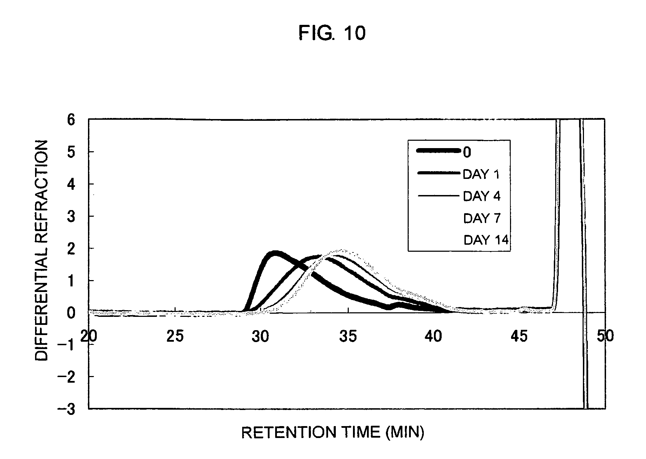

A result of chromatography when HYAL1 was used as the enzyme source and HA as the substrate is shown in Fig. 8, a result when HYAL1 was used as the enzyme source and CSA as the substrate is shown in Fig. 9, and a result when HYAL1 was used as the enzyme source and CSC as the substrate is shown in Fig. 10. Also, a result when PH20 was used as the enzyme source and HA as the substrate is shown in Fig. 11, and a result when PH20 was used as the enzyme source and CSA as the substrate is shown in Fig. 12. The vertical axis of each graph shows differential refraction, and the horizontal axis shows time (retention time; min). In the drawing, 0 day, 1 day, 4 day, 7 day and 14 day indicate reaction times of respective samples. Also, the thickest solid line (black line) in the drawing shows a result on the 0 day, the second thick solid line (red line) shows that on the 1 day, the thinnest solid line (blue line) shows that on the 4 day, the thickest dotted line (green line) shows that on the 7 day, and the second thick dotted line (orange line) shows that on the 14 day.

When the retention time of the peak locating at about the center of each graph is shifted to a longer time side similar to the case of (3), it means that the molecular weight of the substrate was decreased.

It was found from Fig. 8 that the retention time of the peak observed when the reaction time was 0 shifted to the longer time side when the reaction was carried out for 1 day or more (molecular weight of HA was decreased). In the same manner, it was found from Fig. 9 that the peak retention time also shifted to the longer time side in the case of the use of CSA as the substrate when the reaction was carried out for 1 day or more, and from Fig. 10 that the same result was obtained when CSC was used as the substrate. Based on this result, it was found that HYAL1 shows almost the same reactivity with HA, CSA and CSC.

It was found based on Fig. 11 that the retention time of the peak shifted to the long time side according to the reaction time when PH20 was used as the enzyme source and HA was used as the substrate. On the other hand, when CSA was used as the substrate, it was shown by Fig. 12 that the retention time of the peak does not show a significant change even when the reaction time is prolonged. Based on this, it was found that PH20 shows stronger reactivity with HA than reactivity with CSA.

Based on the above results, it was found that HYAL1 has a substrate reactivity which is different from that of PH20.

While the present invention has been described in detail and with reference to specific embodiments thereof, it will be apparent to one skill in the art that various changes and modifications can be made therein without departing from the spirit and scope thereof.

This application is based on Japanese patent applications No. 2003-097301 filed on March 31, 2003 and No. 2003-113965 filed on April 18, 2003, the entire contents of which are incorporated hereinto by reference. All references cited herein are incorporated in their entirety.

### INDUSTRIAL APPLICABILITY

Since the catalyst of the present invention and the cleavage agent of the present invention are capable of specifically cleaving one sugar chain or two or more sugar chains selected from the group consisting of HA, CSA, CSC and CSD, they are markedly useful because of their capability of specifically cleaving such sugar chains (decreasing the molecular weight). Since the medicament of the present invention can specifically cleave one sugar chain or two or more sugar chains selected from the group consisting of HA, CSA, CSC and CSD distributing in a living body tissue, the medicament is markedly useful because of its capability of cleaving such sugar chains which are desired to be cleaved and to retain sugar chains which should not be cleaved. In addition, the medicament of the present invention (the catalyst of the present invention, the cleavage agent of the present invention) is markedly useful, since these are capable of cleaving one sugar chain or two or more sugar chains selected from the group consisting of HA, CSA, CSC and CSD by hydrolysis, therefore unsaturated products are not formed, different from the case of bacterial lyase enzyme and there is a possibility of making into a medicament having further higher safety.

In addition, the cleavage method of the present invention is also markedly useful, because it can specifically cleave one sugar chain or two or more sugar chains selected from the group consisting of HA, CSA, CSC and CSD.

The production method of the sugar chain of the present invention is also markedly useful, because it can produce predetermined sugar chains having a decreased molecular weight specifically, conveniently, quickly, massively and inexpensively.

The production method of the catalyst of the present invention, the cleavage agent producing method of the present invention and the medicament producing method of the present invention are also markedly useful, because they can produce the catalyst of the present invention, the cleavage agent of the present invention and the medicament of the present invention conveniently, quickly, massively and inexpensively. The fusion protein of the present invention is also markedly useful, because it can be used as an essential component of the catalyst of the present invention, the cleavage agent of the present invention and the medicament of the present invention.

## Claims

1. A catalyst which comprises the following protein (A) or (B) as an active ingredient and is capable of specifically cleaving one sugar chain or two or more sugar chains selected from the group consisting of hyaluronic acid, chondroitin sulfate A, chondroitin sulfate C and chondroitin sulfate D:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

2. The catalyst according to claim 1, which is not capable of cleaving dermatan sulfate, keratan sulfate, and chondroitin having an average molecular weight of 7,000.

3. A cleavage agent which comprises the following protein (A) or (B) as an active ingredient and is specific for cleavage of one sugar chain or two or more sugar chains selected from the group consisting of hyaluronic acid, chondroitin sulfate A, chondroitin sulfate C and chondroitin sulfate D:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

4. The cleavage agent according to claim 3, which is not capable of cleaving dermatan sulfate, keratan sulfate, and chondroitin having an average molecular weight of 7,000.

5. A medicament which comprises the following protein (A) or (B) as an active ingredient and is used for specifically cleaving one sugar chain or two or more sugar chains selected from the group consisting of hyaluronic acid, chondroitin sulfate A, chondroitin sulfate C and chondroitin sulfate D which are present in a living body tissue:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

6. The medicament according to claim 5, which is not capable of cleaving dermatan sulfate, keratan sulfate, and chondroitin having an average molecular weight of 7,000.

7. The medicament according to claim 5 or 6, wherein the living body tissue is nucleus pulposus.

8. The medicament according to any one of claims 5 to 7, which is an agent for treating disc herniation.

9. A method for specifically cleaving one sugar chain or two or more sugar chains selected from the group consisting of hyaluronic acid, chondroitin sulfate A, chondroitin sulfate C and chondroitin sulfate D, which comprises reacting the following protein (A) or (B) with the sugar chain(s):
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

10. The method according to claim 9, wherein the protein is not capable of cleaving dermatan sulfate, keratan sulfate, and chondroitin having an average molecular weight of 7,000.

11. A method for specifically producing a sugar chain(s) having a decreased molecular weight, which comprises reacting the following protein (A) or (B) with one sugar chain or two or more sugar chains selected from the group consisting of hyaluronic acid, chondroitin sulfate A, chondroitin sulfate C and chondroitin sulfate D:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

12. The method according to claim 11, wherein dermatan sulfate, keratan sulfate and chondroitin having a decreased molecular weight are not produced.

13. A method for producing the catalyst according to claim 1 or 2, which comprises expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

14. A method for producing the cleavage agent according to claim 3 or 4, which comprises expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

15. A method for producing the medicament according to any one of claims 5 to 8, which comprises expressing a protein using a DNA comprising the following (a) or (b); and collecting the expressed protein:
(a) a DNA encoding a protein which consists of the amino acid sequence represented by SEQ ID NO: 2;
(b) a DNA encoding a protein which consists of an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

16. A fusion protein which comprises the following protein (A) or (B) with other peptide:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

17. The fusion protein according to claim 16, which is not capable of cleaving dermatan sulfate, keratan sulfate, and chondroitin having an average molecular weight of 7,000.

18. A method for treating a disease in which one sugar chain or two or more sugar chains selected from the group consisting of hyaluronic acid, chondroitin sulfate A, chondroitin sulfate C and chondroitin sulfate D is/are excessively present in a living body tissue, which comprises administering the medicament according to claim 5.

19. The method according to claim 18, wherein the medicament is not capable of cleaving dermatan sulfate, keratan sulfate, and chondroitin having an average molecular weight of 7,000.

20. The method according to claim 18, wherein the living body tissue is nucleus pulposus.

21. The method according to claim 18, wherein the disease is disc herniation.

22. Use of the following protein (A) or (B) as a catalyst which is capable of specifically cleaving one sugar chain or two or more sugar chains selected from the group consisting of hyaluronic acid, chondroitin sulfate A, chondroitin sulfate C and chondroitin sulfate D:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

23. Use of the following protein (A) or (B) as a cleavage agent which is specific for cleavage of one sugar chain or two or more sugar chains selected from the group consisting of hyaluronic acid, chondroitin sulfate A, chondroitin sulfate C and chondroitin sulfate D:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

24. Use of the following protein (A) or (B) for the manufacture of a medicament which is capable of specifically cleaving one sugar chain or two or more sugar chains selected from the group consisting of hyaluronic acid, chondroitin sulfate A, chondroitin sulfate C and chondroitin sulfate D which are present in a living body tissue:
(A) a protein which comprises the amino acid sequence represented by SEQ ID NO: 2;
(B) a protein which comprises an amino acid sequence having deletion, substitution, insertion or transposition of one or several amino acids in the amino acid sequence represented by SEQ ID NO: 2, and has activity capable of specifically cleaving one sugar chain or two or more sugar chains selected from the group consisting of hyaluronic acid, chondroitin sulfate A, chondroitin sulfate C and chondroitin sulfate D.

25. The use according to claim 24, wherein the medicament is not capable of cleaving dermatan sulfate, keratan sulfate, and chondroitin having an average molecular weight of 7,000.

26. The use according to claim 24 or 25, wherein the living body tissue is nucleus pulposus.

27. The use according to any one of claims 24 to 26, wherein the medicament is for treating disc herniation.


SEQUENCE LISTING

<110>  Seikagaku Corporation

<120>  Catalyst for cleaving a sugar chain

<130>  P04778600

<150>  JP 2003-097301
<151>  2003-03-31

<150>  JP 2003-113965
<151>  2003-04-18

<160>  2
<170>  PatentIn version 3.1

<210>  1
<211>  1308
<212>  DNA
<213>  Homo sapiens

<220>
<221>  CDS
<222>  (1)..(1308)
<223>

<400>  1
atg gca gcc cac ctg ctt ccc atc tgc gcc ctc ttc ctg acc tta ctc      48
Met Ala Ala His Leu Leu Pro Ile Cys Ala Leu Phe Leu Thr Leu Leu
1               5                   10                  15
gat atg gcc caa ggc ttt agg ggc ccc ttg cta ccc aac cgg ccc ttc      96
Asp Met Ala Gln Gly Phe Arg Gly Pro Leu Leu Pro Asn Arg Pro Phe
            20                  25                  30
acc acc gtc tgg aat gca aac acc cag tgg tgc ctg gag agg cac ggt      144
Thr Thr Val Trp Asn Ala Asn Thr Gln Trp Cys Leu Glu Arg His Gly
        35                  40                  45
gtg gac gtg gat gtc agt gtc ttc gat gtg gta gcc aac cca ggg cag      192
Val Asp Val Asp Val Ser Val Phe Asp Val Val Ala Asn Pro Gly Gln
    50                  55                  60
acc ttc cgc ggc cct gac atg aca att ttc tat agc tcc cag ctg ggc      240
Thr Phe Arg Gly Pro Asp Met Thr Ile Phe Tyr Ser Ser Gln Leu Gly
65                  70                  75                  80
acc tac ccc tac tac acg ccc act ggg gag cct gtg ttt ggt ggt ctg      288
Thr Tyr Pro Tyr Tyr Thr Pro Thr Gly Glu Pro Val Phe Gly Gly Leu
                85                  90                  95

ccc cag aat gcc agc ctg att gcc cac ctg gcc cgc aca ttc cag gac 336
Pro Gln Asn Ala Ser Leu Ile Ala His Leu Ala Arg Thr Phe Gln Asp
            100                 105                 110
atc ctg gct gcc ata cct gct cct gac ttc tca ggg ctg gca gtc atc 384
Ile Leu Ala Ala Ile Pro Ala Pro Asp Phe Ser Gly Leu Ala Val Ile
        115                 120                 125
gac tgg gag gca tgg cgc cca cgc tgg gcc ttc aac tgg gac acc aag 432
Asp Trp Glu Ala Trp Arg Pro Arg Trp Ala Phe Asn Trp Asp Thr Lys
    130                 135                 140
gac att tac cgg cag cgc tca cgg gca ctg gta cag gca cag cac cct 480
Asp Ile Tyr Arg Gln Arg Ser Arg Ala Leu Val Gln Ala Gln His Pro
145                 150                 155                 160
gat tgg cca gct cct cag gtg gag gca gta gcc cag gac cag ttc cag 528
Asp Trp Pro Ala Pro Gln Val Glu Ala Val Ala Gln Asp Gln Phe Gln
                165                 170                 175
gga gct gca cgg gcc tgg atg gca ggc acc ctc cag ctg ggg cgg gca 576
Gly Ala Ala Arg Ala Trp Met Ala Gly Thr Leu Gln Leu Gly Arg Ala
            180                 185                 190
ctg cgt cct cgc ggc ctc tgg ggc ttc tat ggc ttc cct gac tgc tac 624
Leu Arg Pro Arg Gly Leu Trp Gly Phe Tyr Gly Phe Pro Asp Cys Tyr
        195                 200                 205
aac tat gac ttt cta agc ccc aac tac acc ggc cag tgc cca tca ggc 672
Asn Tyr Asp Phe Leu Ser Pro Asn Tyr Thr Gly Gln Cys Pro Ser Gly
    210                 215                 220
atc cgt gcc caa aat gac cag cta ggg tgg ctg tgg ggc cag agc cgt 720
Ile Arg Ala Gln Asn Asp Gln Leu Gly Trp Leu Trp Gly Gln Ser Arg
225                 230                 235                 240
gcc ctc tat ccc agc atc tac atg ccc gca gtg ctg gag ggc aca ggg 768
Ala Leu Tyr Pro Ser Ile Tyr Met Pro Ala Val Leu Glu Gly Thr Gly
                245                 250                 255
aag tca cag atg tat gtg caa cac cgt gtg gcc gag gca ttc cgt gtg 816
Lys Ser Gln Met Tyr Val Gln His Arg Val Ala Glu Ala Phe Arg Val
            260                 265                 270
gct gtg gct gct ggt gac ccc aat ctg ccg gtg ctg ccc tat gtc cag 864
Ala Val Ala Ala Gly Asp Pro Asn Leu Pro Val Leu Pro Tyr Val Gln
        275                 280                 285
atc ttc tat gac acg aca aac cac ttt ctg ccc ctg gat gag ctg gag 912
Ile Phe Tyr Asp Thr Thr Asn His Phe Leu Pro Leu Asp Glu Leu Glu
    290                 295                 300
cac agc ctg ggg gag agt gcg gcc cag ggg gca gct gga gtg gtg ctc 960
His Ser Leu Gly Glu Ser Ala Ala Gln Gly Ala Ala Gly Val Val Leu
305                 310                 315                 320
tgg gtg agc tgg gaa aat aca aga acc aag gaa tca tgt cag gcc atc 1008
Trp Val Ser Trp Glu Asn Thr Arg Thr Lys Glu Ser Cys Gln Ala Ile
                325                 330                 335
aag gag tat atg gac act aca ctg ggg ccc ttc atc ctg aac gtg acc 1056

Lys Glu Tyr Met Asp Thr Thr Leu Gly Pro Phe Ile Leu Asn Val Thr
            340                 345                 350
agt ggg gcc ctt ctc tgc agt caa gcc ctg tgc tcc ggc cat ggc cgc 1104
Ser Gly Ala Leu Leu Cys Ser Gln Ala Leu Cys Ser Gly His Gly Arg
        355                 360                 365
tgt gtc cgc cgc acc agc cac ccc aaa gcc ctc ctc ctc ctt aac cct 1152
Cys Val Arg Arg Thr Ser His Pro Lys Ala Leu Leu Leu Leu Asn Pro
    370                 375                 380
gcc agt ttc tcc atc cag ctc acg cct ggt ggt ggg ccc ctg agc ctg 1200
Ala Ser Phe Ser Ile Gln Leu Thr Pro Gly Gly Gly Pro Leu Ser Leu
385                 390                 395                 400
cgg ggt gcc ctc tca ctt gaa gat cag gca cag atg gct gtg gag ttc 1248
Arg Gly Ala Leu Ser Leu Glu Asp Gln Ala Gln Met Ala Val Glu Phe
                405                 410                 415
aaa tgt cga tgc tac cct ggc tgg cag gca ccg tgg tgt gag cgg aag 1296
Lys Cys Arg Cys Tyr Pro Gly Trp Gln Ala Pro Trp Cys Glu Arg Lys
            420                 425                 430
agc atg tgg tga 1308
Ser Met Trp
        435

<210> 2
<211> 435
<212> PRT
<213> Homo sapiens

<400> 2
Met Ala Ala His Leu Leu Pro Ile Cys Ala Leu Phe Leu Thr Leu Leu
1               5                   10                  15
Asp Met Ala Gln Gly Phe Arg Gly Pro Leu Leu Pro Asn Arg Pro Phe
            20                  25                  30
Thr Thr Val Trp Asn Ala Asn Thr Gln Trp Cys Leu Glu Arg His Gly
        35                  40                  45
Val Asp Val Asp Val Ser Val Phe Asp Val Val Ala Asn Pro Gly Gln
    50                  55                  60
Thr Phe Arg Gly Pro Asp Met Thr Ile Phe Tyr Ser Ser Gln Leu Gly
65                  70                  75                  80
Thr Tyr Pro Tyr Tyr Thr Pro Thr Gly Glu Pro Val Phe Gly Gly Leu
                85                  90                  95
Pro Gln Asn Ala Ser Leu Ile Ala His Leu Ala Arg Thr Phe Gln Asp
            100                 105                 110
Ile Leu Ala Ala Ile Pro Ala Pro Asp Phe Ser Gly Leu Ala Val Ile
        115                 120                 125
Asp Trp Glu Ala Trp Arg Pro Arg Trp Ala Phe Asn Trp Asp Thr Lys
    130                 135                 140

Asp Ile Tyr Arg Gln Arg Ser Arg Ala Leu Val Gln Ala Gln His Pro
145                 150                 155                 160
Asp Trp Pro Ala Pro Gln Val Glu Ala Val Ala Gln Asp Gln Phe Gln
                165                 170                 175
Gly Ala Ala Arg Ala Trp Met Ala Gly Thr Leu Gln Leu Gly Arg Ala
            180                 185                 190
Leu Arg Pro Arg Gly Leu Trp Gly Phe Tyr Gly Phe Pro Asp Cys Tyr
        195                 200                 205
Asn Tyr Asp Phe Leu Ser Pro Asn Tyr Thr Gly Gln Cys Pro Ser Gly
    210                 215                 220
Ile Arg Ala Gln Asn Asp Gln Leu Gly Trp Leu Trp Gly Gln Ser Arg
225                 230                 235                 240
Ala Leu Tyr Pro Ser Ile Tyr Met Pro Ala Val Leu Glu Gly Thr Gly
                245                 250                 255
Lys Ser Gln Met Tyr Val Gln His Arg Val Ala Glu Ala Phe Arg Val
            260                 265                 270
Ala Val Ala Ala Gly Asp Pro Asn Leu Pro Val Leu Pro Tyr Val Gln
        275                 280                 285
Ile Phe Tyr Asp Thr Thr Asn His Phe Leu Pro Leu Asp Glu Leu Glu
    290                 295                 300
His Ser Leu Gly Glu Ser Ala Ala Gln Gly Ala Ala Gly Val Val Leu
305                 310                 315                 320
Trp Val Ser Trp Glu Asn Thr Arg Thr Lys Glu Ser Cys Gln Ala Ile
                325                 330                 335
Lys Glu Tyr Met Asp Thr Thr Leu Gly Pro Phe Ile Leu Asn Val Thr
            340                 345                 350
Ser Gly Ala Leu Leu Cys Ser Gln Ala Leu Cys Ser Gly His Gly Arg
        355                 360                 365
Cys Val Arg Arg Thr Ser His Pro Lys Ala Leu Leu Leu Leu Asn Pro
    370                 375                 380
Ala Ser Phe Ser Ile Gln Leu Thr Pro Gly Gly Gly Pro Leu Ser Leu
385                 390                 395                 400
Arg Gly Ala Leu Ser Leu Glu Asp Gln Ala Gln Met Ala Val Glu Phe
                405                 410                 415
Lys Cys Arg Cys Tyr Pro Gly Trp Gln Ala Pro Trp Cys Glu Arg Lys
            420                 425                 430
Ser Met Trp
        435